# EUROPÄISCHE PATENTSCHRIFT

(11) **EP 2 270 082 B1**
(45) Veröffentlichungstag und Bekanntmachung des Hinweises auf die Patenterteilung: **21.02.2018**
(21) Anmeldenummer: 10181852.4
(22) Anmeldetag: 13.05.2005
(51) Int. Cl.: A61L 15/28, C08J 9/28, A61L 27/20, A61L 27/54, A61L 27/56, A61K 8/73, A61Q 19/00, A61K 8/02, A61L 15/42, A61L 15/44

(54) **ALGINAT-HALTIGE PORÖSE FORMKÖRPERN**
ALGINATE-CONTAINING POROUS SHAPED MOULDINGS
ÉLÉMENTS MOULÉS POREUX À BASE D'ALGINATE

(30) Priorität: 21.05.2004 DE 102004025495
(43) Veröffentlichungstag der Anmeldung: 05.01.2011
(62) Teilanmeldung aus: 05742986.2
(73) Patentinhaber: MedSkin Solutions Dr. Suwelack AG, 48727 Billerbeck (DE)
(72) Erfinder: MALESSA, Ralf, 45134, Essen (DE)
(74) Vertreter: CH Kilger Anwaltspartnerschaft mbB

(56) Entgegenhaltungen:
- EP-A2- 0 202 819
- WO-A-2004/104076
- DE-U1- 20 219 666
- GB-A- 621 230
- PATENT ABSTRACTS OF JAPAN Bd. 014, Nr. 505 (C-775), 5. November 1990 (1990-11-05) & JP 02 208332 A (ASAHI CHEM IND CO LTD), 17. August 1990 (1990-08-17)

## Beschreibung

### STAND DER TECHNIK

Die Erfindung betrifft einen schichtförmigen gefriergetrockneten porösen Formkörper, dessen Länge und Breite wenigstens 10-mal so gross wie dessen Dicke sind, enthaltend Alginat, Calcium, Sulfat sowie Carboxymethylcellulose Hyaluronsäure und/oder deren Salze, der dadurch gekennzeichnet ist, dass er durch ein Verfahren erhältlich ist, umfassend das Mischen einer wässrigen Alginatlösung mit Calciumsulfat in Gegenwart mindestens einer Mineralsäure, Giessen der erhaltenen Mischung in eine Form und trocknen der Mischung
und deren Verwendung.

Es ist bekannt, dass Alkalialginate, wie z.B. Na-Alginat, wasserlöslich, Erdalkali-Alginate, wie z.B. Ca-Alginat, hingegen wasserunlöslich sind. Dünne wasserunlösliche Schichten lassen sich daher beispielsweise durch Besprühen eines dünnen Na-Alginatfilmes mit einer CaCl₂-Lösung herstellen. Will man hingegen dickerer Schichten herstellen, so besteht die Schwierigkeit darin, dass die homogene Einarbeitung freier Ca-Ionen in eine Na-Alginatlösung durch eine starke Zunahme der Lösungsviskosität erschwert wird, so dass keine gleichmäßigen Produkte, sondern unzusammenhängende Ca-Alginat-Agglomerate entstehen.

Um dieses Problem zu überkommen schlägt die US 5718916 beispielsweise vor, die wässrige Lösung der wasserlöslichen AlginatZusammensetzung mit einem wasserlöslichen Komplexierungsmittel, wie z.B. Natriumzitrat zu versetzen. Wird anschließend beispielsweise ein leicht lösliches Calciumsalz wie Calciumchlorid hinzugegeben, so wird durch die Anwesenheit des Komplexierungsmittels die sofortige Ausfällung von Calciumalginat verzögert, wodurch die Bildung von unlöslichen Calciumalginat-Kügelchen in dem Produkt verhindert werden soll. In den Beispielen der genannten US-Patentschrift wird jedoch in Maßstäben von wenigen Millilitern gearbeitet. Die Gelierzeit der Alginatlösung nach Zusatz des Calciumchlorids beträgt lediglich 30 bis 60 Sekunden. Versucht man dieses Verfahren auf größere Maßstäbe zu übertragen, so stellt sich heraus, dass die gewünschte Verzögerung durch Zugabe des Komplexierungsmittels zur Lösung des Natriumalginates nicht ausreichend ist, und ein relativ großformatiges Produkt mit einer hohen Homogenität nicht erhalten werden kann. Weiterhin ist bei dem genannten Verfahren die Anwendung von oberflächenaktiven Mitteln obligatorisch, um eine ausreichende Dispergierung der Komponenten zu erzielen. Die Verwendung derartiger oberflächenaktiver Mittel kann jedoch zu Unverträglichkeiten beispielsweise bei der Anwendung auf der Haut führen. Die Tatsache, dass in dem Verfahren der US 5718916 keine ausreichende Verzögerung der Fällung durch die vorherige Zugabe des Komplexierungsmittels erzielt wird, wird auch in der GB 235 7 7 65 desselben Erfinders bestätigt, worin das Verfahren der US 5718916 folglich als nachteilig beschrieben wird. Die GB 2357765 offenbart ein Verfahren zur Herstellung von wasserunlöslichen Alginatschwämmen oder Schaumprodukten für die Herstellung von Wundpflastern oder chirurgischen Produkten, bei dem ebenfalls wasserlösliches Alginat durch den Zusatz mehrwertiger Metallionen in Anwesenheit eines schaumbildenden Mittels vernetzt wird. Auf die Anwesenheit eines Komplexierungsmittels wird dabei bewusst verzichtet. In einer bevorzugten Variante wird in Gegenwart von Ammoniumhydroxid gearbeitet, um die Viskosität des Calciumalginates herabzusetzen. In den Beispielen wird beispielsweise Calciumsulfat und anschließend Essigsäure zugesetzt. Das Verfahren erfordert die Anwesenheit eines Schaumbildners, oberflächenaktiver Mittel, eines Boratpuffers sowie die erwähnten Ammoniumverbindungen. Diese komplexe Stoffmischung macht das Verfahren schwer steuerbar, und die erhaltenen Produkte enthalten eine Vielzahl von Komponenten, deren physiologische Wirkungen berücksichtigt werden müssen.

Aus der DE 202 19 666 U1 und EP 0 202 819 A2 sind Auflagen für dermatologische Anwendungen, enthaltend ein Trägermaterial auf Polymerbasis, insbesondere Alginsäure-Basis beschrieben. Konkrete Beispiele zur Herstellung dieser Auflagen in Gegenwart mindestens einer Mineralsäure sind diesen Dokumenten nicht zu entnehmen.

Das Dokument WO 2004/104076 offenbart einen gefriergetrockneten porösen Formkörper aus Alginat und Calciumsulfat und gegebenenfalls Carboxymthylcellulose oder Hyaluronsäure oder deren Salze. Eine Mischung von Alginat mit Claciumsulfat in Gegenwart einer Mineralsäure für die Herstellung des Formkörpers wird nicht gelehrt.

Weiterhin offenbart die DE 43 28 329 gefriergetrocknete Biomatrices zur Hautbefeuchtung und zur topischen transdermalen Applikation von pharmazeutischen kosmetisch aktiven Wirkstoffen, die natürliche Polysaccharide und modifizierte Polysaccharide enthalten.
Auch erwähnt diese Schrift bereits die Stabilisierung der Biomatrix durch Bildung von Calciumalginatgerüsten durch Zusatz von Calciumionen. Wie man homogene dickere Alginatschichten herstellt, lässt sich dieser Druckschrift nicht entnehmen.

Die Herstellung kleinformatiger Alginatschwämme für die orale Einnahme durch Versetzen von Na-Alginatlösung mit einem löslichen Calciumsalz (Calciumgluconat) ist in der WO 01/17377 beschrieben. Dieses Verfahren ist jedoch für die Herstellung großformatiger Alginatschwämme aus den vorstehend bereits erwähnten Gründen (keine homogene Einarbeitung der Calciumionen) ungeeignet. Durch die entstehenden Inhomogenitäten ist weiterhin die dort vorgeschlagene Beaufschlagung mit Wirkstoffen erschwert.

Aus der WO94/00512 ist ein Verfahren zur Bildung von Polysaccharidschäumen bekannt, insbesondere auf Alginatbasis. Diese Patentschrift offenbart in einer Ausführungsform auch die Variante, bei der ein unlösliches Carbonat oder Hydrogencarbonatsalz von mehrwertigen Metallkationen in dem geschäumten Polysaccharid dispergiert werden, und der Schaum nachfolgend mit einer starken Säure behandelt wird, um Kohlendioxid freizusetzen, und durch die sich bildenden Kationen das Polysaccharid unter Bildung einer dimensionsstabilen Schaumstruktur zu vernetzen. Auf diese Weise lassen sich nach Angaben in der Druckschrift Schaumdicken von bis zu 5 mm stabilisieren. Diese Dicken sind jedoch insbesondere dann unzureichend, wenn es gewünscht ist, die Schaumformkörper anschließend in dünnere Schichten zu schneiden. Außerdem führt die Verwendung des Calciumcarbonates zu einer (erwünschten) Gasbildung während der Herstellung dazu, dass sich die Porengrößen kaum kontrollieren lassen und starke Inhomogenitäten im Schaum resultieren.

Ein weiteres Verfahren zur Herstellung von Alginatschwämmen ist aus der US 3653383 bekannt. Dabei wird zunächst Ca-Alginat aus Alginsäure und Calciumcarbonat hergestellt, das entstandene Ca-Alginat anschließend zerkleinert und das resultierende Gel der Gefriertrocknung unterworfen. Auf diese Weise können zwar relativ großformatige schwammartige Materialien hergestellt werden, die erhaltenen Produkte zerfallen jedoch in Wasser relativ rasch. Die Alginatschwämme besitzen daher - insbesondere auch wenn sie in dünne Schichten geschnitten werden - eine für kosmetische oder medizinische Auflagen unzureichende Nassfestigkeit, insbesondere Nassreißfestigkeit,

In der Deutschen Patentanmeldung DE 10323794.1 wird ein Verfahren zur Herstellung poröser Alginatformkörper beschrieben, dass den Zusatz von Komplexbildnern für mehrwertige Metallionen bzw. eines Salzes eines mehrwertigen Metallions mit einem mehrzähnigen komplexierenden Anion erfordert. Die Verwendung von Calciumsulfat und einer Mineralsäure bei der Herstellung der porösen Alginatformkörper wird nicht gelehrt.

Die Aufgabe der vorliegenden Erfindung bestand somit in der Bereitstellung von relativ großformatigen, hoch homogenen Formkörpern auf der Basis von Verbindungen zwischen Alginaten und mehrwertigen Metallionen, die über eine hohe Nassfestigkeit, insbesondere über eine hohe Nassreißfestigkeit verfügen, die mit üblichen Schneidvorrichtungen in dünne Schichten geschnitten werden können, die optisch ansprechend sind, d.h. insbesondere eine hohen Weißgrad aufweisen, und die somit in kosmetischen oder medizinischen Anwendungen, wie als kosmetische Hautauflage oder als medizinische Wundauflage verwendet werden können. Weiterhin sollte das Verfahren zur Herstellung der Formkörper einfach zu steuern sein, und auf physiologisch nicht unbedenkliche Zusätze wie Schaumbildner, oberflächenaktive Mittel, Boratpuffer sowie Ammoniumverbindungen möglichst verzichten.

Weiterhin sollte es die Bereitstellung homogener dicker poröser Alginat-Schichten ermöglichen, aus denen in einfacher Weise durch Komprimieren und/oder Ausstanzen geeignete, auch oral verabreichbare kosmetische oder medizinische Applikationsformen, wie z.B. Implantat-Formkörper, Sättigungskomprimate, Mittel zur kontrollierten, insbesondere verzögerten Wirkstofffreisetzung oder dergleichen herzustellen.

Den Erfindern der vorliegenden Patentanmeldung gelang es überraschend, homogene, relativ dicke, großformatige Formkörper auf der Basis von Alginaten mehrwertiger Metallsalzen bereitzustellen, die durch das spezielle, ebenfalls den Gegenstand dieser Erfindung bildende Verfahren erhalten werden können, welche die obigen Probleme der Formkörper aus dem Stand der Technik lösen, und die sich daher hervorragend für die Herstellung kosmetischer oder medizinischer Produkte eignen. Die Formkörper enthalten bevorzugt weder Schaumbildner, noch oberflächenaktive Mittel, noch Boratpuffer, noch Ammoniumverbindungen.

### DETAILLIERTE BESCHREIBUNG DER ERFINDUNG

Das vorliegende Patent stellt somit ein Verfahren zur Herstellung von Alginat-haltigen porösen Formkörpern bereit, dass das Mischen einer wässrigen Alginatlösung mit Calciumsulfat in Gegenwart mindestens einer Mineralsäure, dass Giessen der erhaltenen Mischung in eine Form und das Trocknen der Mischung umfasst. Mineralsäuren schließen beispielsweise Salzsäure, Schwefelsäure und Phosphorsäure ein. Bevorzugt ist Salzsäure.

Die erfindungsgemäß verwendeten wasserlöslichen Alginate sind bevorzugt Alkalimetallalginate, wie Alginate von Natrium, Kalium

Die zugrundeliegende Alginsäure ist ein natürliches saures Polysaccharid, das vor allem aus sogenannten Braunalgen (Phaecophyceae) mit einem hohen, von etwa 30 000 bis 200 000 Dalton schwankenden Molekulargewicht extrahiert wird und Ketten enthält, die aus D-Mannuron- und L-Guluronsäure gebildet werden. Der Polymerisationsgrad ändert sich in Abhängigkeit von der Art der zur Extraktion verwendeten Alge, der Jahreszeit, in der die Algen gesammelt wurden, und dem Ursprungsort der Algen, sowie dem Alter der Pflanzen. Die Hauptarten der Braunalgen, aus denen Alginsäure erhalten wird, sind beispielsweise Macrocystis pyrifera, Laminaria cloustoni, Laminaria hyperborea, Laminaria flexicaulis, Laminaria digitata, Ascophyllumnodosum und Fucus serratus. Alginsäure oder alkalische Alginate können jedoch auch mikrobiologisch, beispielsweise durch Fermentation mit Pseudomonas aeruginosa oder Mutanten von Pseudomonas putida, Pseudomonas fluorescens oder Pseudomonas mendocina, erhalten werden (s. z.B. EP-A-251905 und Römpp Chemie Lexikon "Naturstoffe" Thieme Verlag, 1997 und dort zitierte Dokumente).

Erfindungsgemäß bevorzugt sind Alginate mit einer durchschnittlichen Partikelgröße von bis zu etwa 0,2 mm und einer Viskosität in wässriger Lösung (1-%ige Lösung, pH 7, 20°C von 300 bis 800 mPas.

Erfindungsgemäß besonders bevorzugt ist Natriumalginat.

Die verwendete wässrige Lösung des wasserlöslichen Alginates weist bevorzugt eine solche Konzentration auf, dass sich in der nach Zugabe des Calciumsulfates und der Mineralsäure gebildeten wässrigen Suspension eine Konzentration von 0,2 bis 3,0 %, bevorzugter 0,3 bis 2,5-%, noch bevorzugter 0,4 bis 1,2 % (Gew./Gew.) Alginat bezogen auf die eingesetzte Wassermenge einstellt. Die Lösung kann hergestellt werden durch Suspendieren der gewünschten Alginatmenge beispielsweise in destilliertem Wasser. Die Konzentration des Alginates in der wässrigen Suspension hat Einfluss auf die Härte der gebildeten poröse Formkörper. Konzentrationen von mehr als 2 % (w/w) führen zu relativ harten bzw. spröden Formkörpern, was weniger bevorzugt ist. Kleinere Konzentrationen als 2 % (w/w) führen zu weniger spröden Formkörpern, was bevorzugter ist.

In einer weiteren bevorzugten Ausführungsform enthalten die erfindungsgemäßen porösen Formkörper Carboxymethylcellulose, insbesondere Natriumcarboxymethylcellulose. Der Zusatz von Natriumcarboxymethylcellulose führt überraschend zu einer Verbesserung der optischen Dichte der erfindungsgemäßen porösen Formkörper ohne dabei die Härte oder die Sprödheit der Formkörper zu erhöhen. Im Gegenteil führt der Zusatz von Natriumcarboxymethylcellulose, zu einer Verbesserung der Flexibilität der erhaltenen porösen Formkörper. Weiterhin führt der Zusatz der Carboxymethylcellulose, insbesondere Natriumcarboxymethylcellulose, zu einer Stabilisierung der Formkörper. Bei der Herstellung Carboxymethylcellulose-haltiger Formkörper verhindert die Carboxymethylcellulose, insbesondere Natriumcarboxymethylcellulose, überraschend auch eine Sedimentation des schwerlöslichen Salzes, insbesondere des CaSO₄ und erlaubt somit dessen homogenere Einarbeitung in die wässrige Suspension und einer Erhöhung der Homogenität der erhaltenen Formkörper. In einer bevorzugten Ausführungsform des erfindungsgemäßen Verfahrens bildet man daher eine Aufschlämmung des CaSO₄ und der Natriumcarboxymethylcellulose in Wasser, und versetzt diese mit der wässrigen Natriumalginatlösung, die die Mineralsäure und gegebenenfalls weitere Inhaltsstoffe, wie weiter unten beschrieben enthält.

Die Carboxymethylcellulose, insbesondere Natriumcarboxymethylcellulose, kann in den erfindungsgemäßen Formkörpern in einer Menge von bis zu 90 Gew.-% bezogen auf den Trockengehalt des Formkörpers vorliegen. Dies entspricht einzustellenden Vorzugsbereichen in der wässrigen Suspension von etwa bis zu 3 Gew.-%, bevorzugt 0,2 bis 3 Gew.-%.

Eine bevorzugte Ausführungsform des erfindungsgemäßen Formkörpers enthält Carboxymethylcellulose, insbesondere Natriumcarboxymethylcellulose, und Hyaluronsäure bzw. deren Salze oder Derivate.

In dem erfindungsgemäßen Verfahren ist es prinzipiell auch möglich einen Komplexbildner für Calcium zuzusetzen, um die Konzentration der Calciumionen in der Lösung herabzusetzen und somit die Vernetzung des Alginates zu inhibieren, obwohl dies nicht zwingend erforderlich ist. Ein solcher Komplexbildner kann ein Carboxylat einer α-Hydroxypolycarbonsäure, wie ein Zitrat oder Malat, sein, das jedoch als kosmetisch wirksamer Bestandteil, wie als Hautbefeuchter dienen kann.

Erfindungsgemäß zeigt sich überraschend, dass der durch die Mineralsäure eingestellte pH-Wert, einen Einfluß auf die Reißfestigkeit der erhaltenen porösen Formkörper besitzt. Um eine höhere Reißfestigkeit zu erzielen, ist daher ein pH-Wert von weniger als 6 bevorzugt, bevorzugter von weniger als 5. Diese niedrigen pH-Werte sind wiederum besonders bevorzugt in Kombination mit einer niedrigen Alginatkonzentration von weniger als 2 % (w/w) in der erhaltenen Suspension.

Die Menge des CaSO₄ wird zweckmäßig so gewählt, dass die Konzentration des Salzes in der resultierenden Suspension von etwa 0,1 bis 500 mmol/Liter beträgt, wobei hier die Gesamtmenge des Salzes bezogen auf das Volumen der Suspension gemeint ist.

Die Menge des zugegebenen CaSO₄ bezogen auf die Menge des löslichen Alginates in der Lösung wird bevorzugt so gewählt, dass das molare Verhältnis des Alginates zum CaSO₄ von 0,001 bis 1 beträgt.

Die Bildung der schwerlöslichen Alginate wird zweckmäßig so gesteuert, dass mindestens etwa 1 Minute, bevorzugt etwa 2 Minuten noch bevorzugter mindestens etwa 3 Minuten eine Fließfähigkeit der Alginatlösung ausgedrückt als Viskosität bei Raumtemperatur (20°C) von unter etwa 1000 mPas ermöglicht wird.

Das Zusammenmischen von wässriger Alginatlösung, Calciumsulfat und mindestens einer Mineralsäure kann bevorzugt in Mischern mit einem Stator-/Rotorsystem, z.B. in einer Kolloidmühle erfolgen.

Erfindungsgemäß wird die (noch) fließfähige Alginatzusammensetzung in eine für die spätere Trocknung gewünschte Form gegossen. Dabei sind Schichtdicken der fließfähigen Alginatzusammensetzung von bis zu etwa 50 cm möglich. Bevorzugte Formen sind Kastenformen mit rechteckigem Grundriss. Das Giessen kann in jeder geeigneten Stufe des Verfahrens erfolgen. So kann bereits die Lösung des wasserlöslichen Alginates in die spätere für die Trocknung verwendete Form gegossen werden, wenn eine ausreichende Durchmischung in dieser Form gewährleistet werden kann. Bevorzugt erfolgt jedoch das Giessen nach dem die Vernetzung bzw. Fällung des schwerlöslichen Alginates in Gang gesetzt wurde.

Die Trocknung der in die Form gegossenen wässrigen Alginatsuspension erfolgt in an sich bekannter Weise. Besonders bevorzugt ist die Gefriertrocknung. Diese kann ebenfalls in an sich bekannter Weise erfolgen, und hier kann beispielsweise verwiesen werden auf die DE 4328329 C2 oder die DE 4028622 C2, auf die bezüglich des Trocknungsschrittes des erfindungsgemäßen Verfahrens ausdrücklich Bezug genommen werden soll und die somit Teil des erfindungsgemäßen Verfahrens sind.

In einer bevorzugten Ausführungsform des erfindungsgemäßen Verfahrens erfolgt zusätzlich vor dem Giessen der Suspension in eine Form das Zugeben mindestens einer weiteren Komponente, ausgewählt aus der Gruppe, die besteht aus: kosmetischen oder medizinischen Wirkstoffen, weiteren natürlichen oder synthetischen Hydrokolloid-bildenden Polymeren und kosmetischen oder medizinischen Hilfs- bzw. Zusatzstoffen.

Weitere natürliche oder synthetische Hydrokolloid-bildende Polymere schließen (teilweise) wasserlösliche, natürliche oder synthetische Polymere ein, die in wässrigen Systemen Gele bzw. viskose Lösungen bilden. Sie werden zweckmäßig ausgewählt aus weiteren natürlichen Polysacchariden, synthetisch modifizierten Derivaten davon oder synthetischen Polymeren. Weitere Polysaccharide schließen beispielsweise Homoglykane oder Heteroglykane ein, wie zum Beispiel Carrageen, Pektine, Tragant, Guar-Gummi, Johannisbrotkernmehl, Agar-Agar, Gummi-Arabikum, Xanthan, natürliche und modifizierte Stärken, Dextrane, Dextrin, Maltodextrine, Chitosan, Glucane, wie β-1,3-Glucan, β-1,4-Glucan, wie Cellulose, Mucopolysaccharide, wie insbesondere Hyaluronsäure. Synthetische Polymere schließen beispielsweise ein: Celluloseether, Polyvinylalkohol, Polyvinylpyrrolidon, synthetische Cellulosederivate, wie Methylcellulose, Carboxycellulose, Carboxymethylcellulose, insbesondere Natriumcarboxymethylcellulose, Celluloseester, Cellulosesether wie Hydroxypropylcellulose, Polyacrylsäure, Polymethacrylsäure, Poly(methylmethacrylat) (PMMA), Polymethacrylat (PMA), Polyethylenglykole etc. Es können auch Mischungen dieser Polymere verwendet werden. Die von Hydrokolloid-bildenden Proteinen, wie z.B. Kollagen ist jedoch nicht bevorzugt, da einige Endverbraucher zunehmend die Anwendung rein pflanzlicher Produkte insbesondere in der Kosmetik vorziehen.

Erfindungsgemäß besonders bevorzugt wird Hyaluronsäure und/oder deren Salze und/oder deren Derivate zusätzlich hinzugesetzt. Hyaluronsäure ist ein hochviskoses natürliches Glucosaminoglycan mit alternierenden β₁₋₃ Glucoronsäure und β₁₋₄-Glucosaminanteilen; ihr Molekulargewicht liegt zwischen 50000 und einigen Millionen. Hyaluronsäure wird häufig als Natriumsalz, z. B. bei der Therapie, hauptsächlich in der Ophthalmologie, Chirurgie und der Kosmetik verwendet. Die Salze der Hyaluronsäure, die mit Alkali-, Erdalkali-, Magnesium-, Aluminium-, Ammonium- oder substituierten Ammoniumionen gebildet werden, können als Träger zur Steigerung der Absorption von Arzneimitteln verwendet werden (s. z.B. Römpp Chemie Lexikon "Naturstoffe" Thieme Verlag, 1997 und dort zitierte Dokumente). Erfindungsgemäß besonders bevorzugt ist Natriumhyaluronat mit einem Molekulargewicht von etwa 1.000.000 bis 2.500.000 Dalton. Der Zusatz der Hyaluronsäure im erfindungsgemäßen Verfahren führt völlig überraschend zu einem erhöhten Weißgrad der erhaltenen Alginat-haltigen porösen Formkörper. Dies ist insbesondere in der kosmetischen Anwendung aus ästhetischen Gründen sehr bevorzugt. Darüber hinaus entfaltet die Hyaluronsäure aber auch insbesondere bei der topischen bzw. äußeren Anwendung ihre therapeutische Wirkung wie z.B. die Befeuchtung der Haut oder die Förderung der Wundheilung,

Die Hyaluronsäure oder deren Salze werden den erfindungsgemäßen Alginat-haltigen porösen Formkörpern in einer Menge bezogen auf den getrockneten Formkörper von etwa 0,1 bis 90 Gew-%, bevorzugt 1 bis etwa 67 Gew.-% zugesetzt.

Erfindungsgemäß zugesetzte Wirkstoffe schließen insbesondere kosmetische oder therapeutische bzw. pharmazeutische, insbesondere für die äußere Anwendung geeignete Wirkstoffe ein. Bevorzugt enthält der erfindungsgemäß hergestellte Formkörper mindestens einen kosmetischen und/oder pharmazeutischen Wirkstoff. Dementsprechend handelt es sich bei den erfindungsgemäß bevorzugten Formkörpern bevorzugt um kosmetische oder therapeutische Mittel, Kosmetische Formkörper bzw. unter Verwendung kosmetischer Wirkstoffe hergestellte Formkörper im Sinne der Erfindung sind im wesentlichen Mittel im Sinne des Lebensmittel- und Bedarfsgegenständegesetzes (LMBG), d.h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei, denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäß hergestellten kosmetischen Formkörpern beispielweise um kosmetische Auflage, wie z.B. Gesichtsmasken, die beispielweise als Hautwasch- und reinigungsmittel, Hautpflegemittel, insbesondere Gesichtshautpflegemittel, Augenkosmetika, Lippenpflegemittel, Nagelpflegemittel, Fußpflegemittel sowie als Haar- oder Zahnpflegemittel dienen können.

Beispiele kosmetisch gegebenenfalls auch z.B. dermatologischer, therapeutisch wirksamer Verbindungen schließen ein: Antiaknemittel, antimikrobielle Mittel, Antitranspirationsmittel, adstringierende Mittel, desodorierende Mittel, Enthaarungsmittel, Konditionierungsmittel für die Haut, hautglättende Mittel, Mittel zur Steigerung der Hauthydratation wie z. B. Glycerin oder Harnstoff, Sonnenschutzmittel, Keratolytika, Radikalfänger für freie Radikale, antiseptische Wirkstoffe, Wirkstoffe zur Behandlung der Anzeichen der Hautalterung und/oder Mittel, welche die Differenzierung und/oder Proliferation und/oder Pigmentierung der Haut modulieren, Vitamine wie Vitamin C, Wirkstoffe mit reizender Nebenwirkung, wie alpha-Hydroxysäuren, β-Hydroxysäuren, alpha-Ketosäuren, β-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, Fettsubstanzen, wie Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Fettsäureester, Ester von Fettalkoholen und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle, wie beispielsweise in der CTFA-Abhandlung, Cosmetic Ingredient Handbook, 1. Auflg., 1988, The Cosmetic, Toiletry and Fragrance Association, Inc., Washington, erwähnt, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, abrasive Mittel.

Weiterhin können Pflanzenwirkstoffextrakte bzw. daraus gewonnene Auszüge oder Einzelstoffe erwähnt werden, die den erfindungsgemäß hergestellten porösen Formkörpern zugesetzt werden können. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B.alkoholische Auszüge, Frischpflanzensaft, Preßsaft), Eleutherokokkus senticosus; Alkaloide: Rauwolfia (z.B. Prajmalin), Immergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wässrig-ethanol, Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte.

Bevorzugte kosmetische Wirkstoffe sind natürliche und synthetische Feuchthaltefaktoren wie z. B. Glycerin, Harnstoff und Ceramide, Hautschutzmittel, Hautaufheller, Vitamine, Antioxidantien, sogenannte Antiagingmittel, antiirritative Mittel, oder Sonnenschutzmittel. Weitere bevorzugte kosmetische Wirkstoffe sind natürliche Fette und Öle, d.h. Triglyceride von natürlichen Fettsäuren, beispielsweise aufgrund ihrer rückfettenden und pflegenden Wirkung auf der Haut.

Ein besonders bevorzugter kosmetischer Wirkstoff ist Harnstoff, von dem man annimmt, dass er auch als Lokalanästhetikum wirkt.

Im Unterschied zu den vorstehend beschriebenen im wesentlichen in der Kosmetik verwendeten Formkörpern handelt es sich bei den therapeutisch verwendeten Formkörpern (Arzneimittel/Medizinprodukte) bevorzugt um solche, die mindestens einen pharmazeutischen bzw. therapeutischen insbesondere auch dermatologischen Wirkstoff enthalten und die im Sinne des Arzneimittelgesetzes unter anderem dazu bestimmt sind, Krankheiten, Leiden, Körperschäden oder krankhafte Beschwerden zu heilen, zu lindern oder zu verhüten. Auch das Alginat selbst kann jedoch auch als solches als pharmazeutisch/therpeutisch wirksamer Bestandteil angesehen werden. Die Mittel bzw. Wirkstoffe sind für die äußere Anwendung bestimmt, wobei es sich um hautaktive Wirkstoffe aber auch um transdermale Wirkstoffe handelt kann. Sie schließen beispielsweise ein: Mittel zur Behandlung von Hautkrankheiten, äußerlich anwendbare Analgetika, z. B. Dextropropoxyphen, Pentazocin, Pethidin, Buprenorphin; Antirheumatika/Antiphlogistika (NSAR), z. B. Indometacin, Diclofenac, Naproxen, Ketoprofen, Ibuprofen, Flurbiprofen, Salicylsäure und -derivate wie Acetylsalicylsäure, Oxicame; Steroidhormone, z. B. Betamethason, Dexamethason, Methylprednisolon, Ethinylestradiol, Medroergotamin, Dihydroergotoxin; Gichtmittel, z. B. Benzbromaron, Allopurinol; Dermatika Externa, einschließlich antibakterielle Mittel, Antimykotika, antivirale Wirkstoffe, entzündungshemmende Wirkstoffe, juckreizstillenden Wirkstoffe, anästhesierende Wirkstoffe, z. B. Benzocain, Corticoide, Aknemittel, antiparasitäre Wirkstoffe; äußerlich anwendbare Hormone; Venentherapeutika; Immunsuppresiva etc. alle für die äußerliche Anwendung.

Bevorzugte therapeutische Mittel sind Analgetika, z.B. Immunsuppressiva, Hormone, Mittel zur Behandlung von Hautkrankheiten, wie der Neurodermitis, der atopischen Dermatitis und Anti-Herpesmittel.

Die erfindungsgemäß hergestellten porösen Formkörper können weiterhin gegebenenfalls einen oder mehrere Hilfsstoffe enthalten, Hilfsstoffe schließen ein: Füllstoffe, pH-Einstellungsmittel, wie Pufferstoffe, Stabilisatoren, Cosolventien, pharmazeutisch und kosmetisch gebräuchliche oder sonstige Farbstoffe und Pigmente, Konservierungsmittel, Weichmacher, Schmiermittel bzw. Gleitmittel. Ein besonders bevorzugter Hilfsstoff stellt das Squalan dar. Das Squalan besitzt eine hautberuhigende und hautglättende Wirkung.

Durch die vorliegende Erfindung lassen sich poröse Formkörper, enthaltend Alginate mehrwertiger Metallionen, die eine Dicke von mindestens einem Zentimeter, bevorzugt mindestens 2 cm aufweisen und die durch Vernetzen (bzw. Ausfällen) von Alginat-haltigen wäßrigen Lösungen mit Salzen mehrwertiger Metallionen und nachfolgender Trocknung der wäßrigen Suspension des erhaltenen, vernetzten Alginats erhalten werden, herstellen. Die Dicke Formkörpers meint dabei den kürzesten Abstand zwischen 2 Punkten in einem solchen Formkörper. Die Herstellung derartig dicker großformatiger Formkörper mit der gewünschten Nassfestigkeit, insbesondere Nassreißfestigkeit Schneidfähigkeit etc, war bisher im Stand der Technik nicht möglich. Diese porösen Formkörper werden bevorzugt nach dem erfindungsgemäßen Verfahren erhalten. Die Verfahren, die die Gefriertrocknung von zerkleinertem unlöslichen Alginaten beinhalteten, führen zu leicht zerfallenden, für die vorliegend anvisierte Verwendung ungeeigneten porösen bzw. schwammartigen Materialien.

Die erfindungsgemäßen porösen Formkörper weisen beim Suspendieren von 1 g des Formkörpers in 100 g Wasser bei 20 °C einen pH-Wert der wässrigen Phase von weniger als 7, bevorzugt weniger als 6 auf. Ein solch saurer pH-Wert ist insbesondere bei der kosmetischen Anwendung auf der Haut bevorzugt.

Der erfindungsgemäße poröse Formkörper weist bevorzugt eine Dichte von 0,005 bis 1 g/cm3, bevorzugt von 0,01 bis 0,5 g/cm3 auf (bestimmt nach DIN 53420).

Der erfindungsgemäße poröse Formkörper weist bevorzugt eine Nassreißfestigkeit von mindestens etwa 10 mN/mm Schichtdicke auf (bestimmt nach DIN 53328).

Der erfindungsgemäße porösen Formkörper bestehen nicht oder im wesentlichen nicht aus gesponnenen Alginatfasern, wie z.B. Calciumalginat-Fasern.

Die genannten erfindungsgemäßen porösen Formkörper können, wie vorstehend erwähnt zusätzlich mindestens eine weitere Komponente enthalten, ausgewählt aus der Gruppe, die besteht aus: kosmetischen oder medizinischen Wirkstoffen, weiteren natürlichen oder synthetischen Hydrokolloid-bildenden Polymeren und kosmetischen oder medizinischen Hilfs- bzw. Zusatzstoffen enthalten. Diese können in den erfindungsgemäßen porösen Formkörpern in Mengen von bis zu 0,75 g / g, bevorzugt weniger als 0,5 g/g des Formkörpers enthalten sein.

Die erfindungsgemäßen porösen Formkörper eignen sich hervorragend zur Herstellung von schichtförmigen Formkörpern durch Schneiden der erfindungsgemäßen porösen Formkörper in an sich bekannter Weise. Dies ist beispielsweise mit den durch Gefriertrocknung zerkleinerter unlöslicher Alginate hergestellten schwammartigen Materialien nicht möglich. Durch das Schneiden der erfindungsgemäßen porösen Formkörper werden beispielsweise Schichtdicken von 0,5 bis 20 mm erhalten, Die Erfindung betrifft auch die so erhaltenen schichtförmigen porösen Formkörper. Derartige schichtartige poröse Formkörper eignen sich insbesondere für die äußere Anwendung, wie als kosmetische oder medizinische Auflage, als Wundverbandmaterial, als Wundauflage, als Implantatmaterial, als Zellzuchtmatrix. Die erfindungsgemäßen porösen Formkörper eignen sich weiterhin auch hervorragend zur Herstellung von komprimierten, expandierbaren, schwammartigen Formkörpern, wie sie z.B. in der EP 0901 792 der Anmelderin auf Kollagenbasis beschrieben sind. Sie lassen sich in einfacher Weise aus den großformatigen insbesondere nach der Gefriertrocknung erhaltenen porösen Formkörpern durch Ausstanzen und/oder Komprimieren insbesondere auch im industriellen Maßstab herstellen, was nach den Methoden des Stand der Technik bislang nicht ohne weiteres möglich ist.

Derartige Komprimate eignen sich insbesondere für die orale, buccale oder nasale Anwendung, wie zum Beispiel als Sättigungskomprimate, die gegebenenfalls zusätzlich Wirkstoffe, Nahrungsergänzungsstoffe oder Vitamine enthalten können (z.B. DE 19942417).

Aufgrund des schwerlöslichen Charakters der erfindungsgemäßen porösen Formkörper eignen sie sich weiterhin zur Herstellung von wirkstoffbeladenen Formen, aus denen der Wirkstoff kontrolliert, insbesondere verzögert freigegeben wird. Derartige Formen schließen sowohl wirkstoffenthaltende Schwämme, wie Implantate, Vaginal-Suppositorien als auch oral verabreichbare Formen, letztere insbesondere als Komprimate, die im befeuchteten Zustand, wie im Magen auf ein mehrfaches ihres komprimierten Volumens expandieren und den enthaltenden Wirkstoff aus der schwammförmigen Matrix freisetzen (z.B. WO 98/09617).

Die vorliegende Erfindung betrifft weiterhin poröse Formkörper, enthaltend Alginate mehrwertiger Metallionen und Hyaluronsäure und/oder deren Salze und/oder deren Derivate, die nach dem erfindungsgemäßen Verfahren erhalten werden. Diese Formkörper verfügen wie oben bereits dargelegt völlig überraschend über einen erhöhten Weißgrad, was insbesondere in der kosmetischen aber auch in der medizinischen Anwendung sehr bevorzugt ist. Bezüglich der Zusammensetzung derartiger Hyaluronsäure-haltiger poröser Formkörper kann beispielsweise auf die obigen Ausführungen verwiesen werden. Die Hyaluronsäure-haltigen porösen Formkörper werden bevorzugt nach dem erfindungsgemäßen Verfahren hergestellt.

Die vorliegende Erfindung betrifft weiterhin die Verwendung der erfindungsgemäßen porösen Formkörpern bzw. der nach dem erfindungsgemäßen Verfahren erhaltenen Formkörper als kosmetische Mittel.

Die Anwendung der erfindungsgemäßen porösen Formkörper in der Kosmetik erfolgt bevorzugt in der Form von kosmetischen Hautauflagen, die befeuchtet auf die Haut aufgetragen und nach einer gewissen Einwirkzeit beispielsweise nach Einziehen der darin enthaltenen Wirkstoffe wieder abgenommen werden. Auch das Alginat selbst entfaltet bereits eine kosmetische Wirkung wie die Hydratation und Glättung der Haut.

Die vorliegende Erfindung betrifft weiterhin die Verwendung der erfindungsgemäßen porösen Formkörpern bzw. der nach dem erfindungsgemäßen Verfahren erhaltenen Formkörper zur Herstellung eines medizinischen Produktes. Derartige medizinische Produkte schließen beispielsweise Wundauflagen, transdermale Auflagen, Wundpflaster, Implantate, Substrate zur Züchtung von Zellen, Mittel für die kontrollierte, insbesondere verzögerte Verabreichung von Wirkstoffen in der Form besagter Implantate, aber auch als oral verabreichbare Retardpräpate, oder als sogenannte Sättigungskomprimate, die durch die Expansion des komprimierten porösen Formkörpers im Magen eine Sättigungswirkung entfalten. Letztere können auch mit Nahrungsergänzungsmitteln, Vitaminen, Mineralien oder sonstigen Wirkstoffen beaufschlagt werden.

Die erfindungsgemäßen porösen Formkörper bzw. die nach dem erfindungsgemäßen Verfahren erhaltenen Formkörper dienen bevorzugt zur äußeren Anwendung, wie insbesondere als kosmetische oder medizinische Auflage. Daneben sind aber wie erwähnt auch beispielsweise die orale, buccale, vaginale, nasale Anwendung möglich. Die erfindungsgemäß zugänglichen homogenen dicken porösen Alginatformkörper erlauben wie gesagt die Herstellung beliebiger dieser Applikationsformen in industriellem Maßstab mit bekannten Methoden, wie Schneiden, Pressen, bzw. Komprimieren und/oder Ausstanzen.

Besonders bevorzugte erfindungsgemäße Formkörper enthalten, bezogen auf die Trockensubstanz also ohne Restfeuchte:
Etwa 6 bis 100 Gew.-% Alginat
0 bis etwa 90 Gew.-% Carboxymethylcellulose, insbesondere deren Natriumsalz,
0 bis etwa 70 Gew.-% Hyaluronsäure bzw. deren Salze oder Derivate,
0 bis etwa 90 Gew.-% natürliche oder synthetische Öle,
0 bis etwa 70 Gew.-% Zitronensäure oder deren Salze,
was Vorzugsgehalten in der zu gefriertrocknenden wässrigen Suspension in Schritt c) von
Etwa 0,2 bis 3 Gew.-% Alginat
0 bis etwa 3 Gew.-% Carboxymethylcellulose, insbesondere deren Natriumsalz,
0 bis etwa 1 Gew.-% Hyaluronsäure bzw. deren Salze oder Derivate,
0 bis etwa 3 Gew.-% natürliche oder synthetische Öle,
0 bis etwa 1 Gew.-% Zitronensäure oder deren Salze,
entspricht.

Die erfindungsgemäßen poröse Formkörper liegen in Form einer Schicht vor, d.h. Länge und Breite des Formkörpers sind wenigstens 10-mal, bevorzugt wenigstens 20-mal so groß wie die Dicke des Formkörpers. Solche Schichten können auch in Formen geschnitten werden, etwa in Form einer Gesichtsmaske. Die Schichten haben eine Fläche von bevorzugt mindestens etwa 25 cm², bevorzugter von mindestens etwa 50 cm², noch bevorzugter von mindestens etwa 100 cm².

Die Erfindung betrifft weiterhin auch Laminate, enthaltend mindestens eine Schicht, wie vorstehend beschrieben, die auf mindestens einer Seite mit mindestens einer weiteren Trägerschicht laminiert ist. Bevorzugt ist die erfindungsgemäße Schicht nur auf einer Seite mit bevorzugt nur einer Trägerschicht laminiert. Die Trägerschicht besteht bevorzugt aus einem Rayonnetz (aus Viskose). Derartige Laminate finden bevorzugt Verwendung als Wundauflage oder Wundpflaster und besonders bevorzugt als kosmetische Maske.

Die Erfindung betrifft auch eine Kombination, enthaltend mindestens einen der erfindungsgemäßen porösen Formkörper sowie mindestens eine wässrige Lösung, die einen oder mehrere Wirkstoffe und/oder Hilfsstoffe enthält, in einer zusammengehörenden, räumlichen Anordnung (Anwendungspaket, Set, Kit-of-Parts) Bei der Wirkstofflösung kann es sich z.B. um Lösungen von leichtflüchtigen Wirk- und/oder Hilfsstoffen handeln, die aufgrund des Herstellverfahrens durch die Gefriertrocknung nicht in den Formkörper eingebracht werden sollen bzw. können, wie z.B. gewisse Anteile ätherischer Öle, Parfums etc. Weiterhin kann die Lösung temperaturempfindliche pharmazeutische oder kosmetische Wirkstoffe enthalten.

Die Offenbarung betrifft insbesondere die folgenden bevorzugten, nicht erfindungsgemäßen, Ausführungsformen:
1. Verfahren zur Herstellung von porösen Formkörpern, dass das Mischen einer wässrigen Alginatlösung mit Calciumsulfat in Gegenwart mindestens einer Mineralsäure, dass Giessen der erhaltenen Mischung in eine Form und das Trocknen der Mischung umfasst.
2. Verfahren noch Ausführungsform 1, worin die Mineralsäure Salzsäure ist.
3. Verfahren nach Ausführungsform 1 oder 2, worin die wässrige Alginatlösung die wässrige Lösung eines Alkalimetallalginates ist.
4. Verfahren nach einer der Ausführungsformen 1 bis 3, worin Carboxymethylcellulose oder ein Salz davon zugesetzt wird.
5. Verfahren nach Ausführungsform 4, worin eine Aufschlämmung von Calciumsulfat und Natrium-Carboxymethylcellulose zugesetzt wird.
6. Verfahren nach einer der Ausführungsformen 1 bis 5, worin ein Komplexbildner für Calcium zugesetzt wird.
7. Verfahren noch einer der Ausführungsformen 1 bis 6, worin ein Carboxylat einer α-Hydroxypolycarbonsäure zugesetzt wird.
8. Verfahren noch einer der Ausführungsformen 1 oder 7, worin ein Zitrat oder Malat zugesetzt wird.
9. Verfahren nach einer der Ausführungsformen 1 bis 8, worin das Trocknen durch Gefriertrocknung erfolgt.
10. Verfahren nach einer der Ausführungsformen 1 bis 9, worin zusätzlich mindestens eine weitere Komponente, ausgewählt aus der Gruppe, die besteht aus: kosmetischen oder medizinischen Wirkstoffen, weiteren natürlichen oder synthetischen Hydrokolloid-bildenden Polymeren und kosmetischen oder medizinischen Hilfs- bzw. Zusatzstoffen vor dem Trocknen zugegeben wird.
11. Verfahren nach Ausführungsform 10, worin die weitere Komponente mindestens ein natürliches Polysaccharid oder ein modifiziertes Derivat davon ist.
12. Verfahren nach Ausführungsform 10 oder 1 1 , worin die weitere Komponente Hyaluronsäure oder ein Salz davon ist.
13. Verfahren nach einer der Ausführungsformen 10 bis 1 2, worin als weitere Komponente Harnstoff zugesetzt wird.
14. Verfahren nach einer der Ausführungsformen 10 bis 13, worin als weitere Komponente Squalan zugesetzt wird.
15. Verfahren nach einer der Ausführungsformen 1 bis 14, worin der Zusatz eines Salzes eines mehrwertigen Metallions mit einem mehrzähnigen komplexierenden Anion ausgenommen ist.
16. Poröser Formkörper, erhältlich nach dem Verfahren nach einer der Ausführungsformen 1 bis 15.
17. Poröser Formkörper, enthaltend Alginat, Calcium, Sulfat und Carboxymethylcellulose.
18. Poröser Formkörper nach Ausführungsform 16 oder 17, der beim Suspendieren von 1 g des Formkörpers in 100 g Wasser bei 20 °C einen pH-Wert der wässrigen Phase von weniger als 7 ergibt.
19. Poröser Formkörper nach einer der Ausführungsformen 1 6 bis 18, der eine Dichte von 0,005 bis 1 g/cm³ nach DIN 53420 aufweist.
20. Poröser Formkörper nach einer der Ausführungsformen 1 6 bis 19, der eine Nassreißfestigkeit nach DIN 53328 von mindestens 10 mN/mm Schichtdicke aufweist.
21. Poröser Formkörper nach einer der Ausführungsformen 1 6 bis 20, der zusätzlich mindestens eine weitere Komponente, ausgewählt aus der Gruppe, die besteht aus: kosmetischen oder medizinischen Wirkstoffen, weiteren natürlichen oder synthetischen Hydrokolloid-bildenden Polymeren und kosmetischen oder medizinischen Hilfs- bzw. Zusatzstoffen enthält.
22. Poröse Formkörper nach einer der Ausführungsformen 1 6 bis 21, enthaltend Hyaluronsäure und/oder deren Salze und/oder deren Derivate.
23. Poröser Formkörper nach einer der Ausführungsformen 16 bis 22, enthaltend mindestens eine Hydroxycarbonsäure,
24. Poröser Formkörper nach einer der Ausführungsformen 1 6 bis 23, enthaltend Harnstoff.
25. Poröser Formkörper nach einer der Ausführungsformen 1 6 bis 24, der Squalan enthält,
26. Verfahren zur Herstellung von schichtförmigen Formkörpern durch Schneiden der Formkörper nach einer der Ausführungsformen 16 bis 25 oder der Formkörper, die nach einer der Ausführungsformen 1 bis 15 erhalten werden.
27. Verfahren zur Herstellung von komprimierten Formkörpern durch Komprimieren der Formkörper nach einer der Ausführungsformen 1 6 bis 25 oder der Formkörper, die nach einer der Ausführungsformen 1 bis 15 erhalten werden.
28. Verfahren nach Ausführungsform 27 zur Herstellung oral verabreichbarer Mittel.
29. Verfahren nach Ausführungsform 27 oder 28 zur Herstellung oral verabreichbarer Sättigungskomprimate.
30. Verfahren nach Ausführungsform 27 oder 28 zur Herstellung oral verabreichbarer Mittel zur kontrollierten Wirkstofffreisetzung.
31. Verwendung von porösen Formkörpern nach einer der Ausführungsformen 16 bis 25 bzw. erhalten nach einer der Ausführungsformen 1 bis 15 als kosmetische Mittel.
32. Verwendung nach Ausführungsform 31, worin das kosmetische Mittel eine kosmetische Auflage, insbesondere eine Gesichtsmaske ist.
33. Verwendung von porösen Formkörpern nach einer der Ausführungsformen 16 bis 25 bzw. erhalten nach einer der Ausführungsformen 1 bis 15 zur Herstellung eines medizinischen Produktes.
34. Verwendung nach Ausführungsform 32 oder 33 zur äußeren, oralen, buccalen, vaginalen oder nasalen Anwendung, oder zur Anwendung als Implantat oder als Wundauflage bzw. Wundpflaster oder in Wirkstoffpflastern.
35. Verwendung nach Ausführungsform 34 zur Herstellung von Implantaten oder Wundauflagen.
36. Poröse Formkörper nach einer der Ausführungsformen 16 bis 25 bzw. erhalten nach einer der Ausführungsformen 1 bis 15, die in Form einer Schicht vorliegen.
37. Laminate, enthaltend mindestens eine Schicht nach Ausführungsform 36, die auf mindestens einer Seite mit mindestens einer weiteren Trägerschicht laminiert sind.
38. Verwendung des Laminates nach Ausführungsform 37 als Wundauflage, Wundpflaster oder kosmetische Auflage, insbesondere Gesichtsmaske.
39. Kombination, enthaltend mindestens einen porösen Formkörpern nach einer der Ausführungsformen 16 bis 25 oder 36, bzw. erhalten nach einer der Ausführungsformen 1 bis 15, sowie mindestens eine wässrige Lösung, die einen oder mehrere Wirkstoffe und/oder Hilfsstoffe enthält, in zusammengehöriger, räumlicher Anordnung.
40. Verwendung der Kombination nach Ausführungsform 39 zur Herstellung eines kosmetischen oder pharmazeutischen Mittels.

Die Erfindung wird im Hinblick auf das folgende Beispiel näher erläutert.

### BEISPIEL 1

### Schritt 1 :

| | |
|---|---|
| 2500 g | RO-Wasser (entsalztes Wasser, Reverse Osmose) |
| 32,5 g | Na-Alginat |
| 10,0 g | Zitronensäure |
| HCl | |

Das Alginatpulver und Zitronensäure mittels eines Mixers ins RO-Wasser einarbeiten bis eine homogene Mischung entsteht. Danach wird HCl zugeben, (In diese Mischung können an dieser Stelle zweckmäßig auch gegebenenfalls kosmetische und/oder medizinische Wirkstoffe und/oder Öle oder sonstige Stoffe eingearbeitet werden).

### Schritt 2:

| | |
|---|---|
| 50 g | RO-Wasser |
| 10,0 g | Calciumsulfat |
| 10,0 g | Natriumcarboxymethylcellulose |

Das Calciumsulfat und Natriumcarboxymethylcellulose wird unter Rühren in 50 ml RO-Wasser gegeben.

### Schritt 3:

Die Lösungen aus Schritt 1 und Schritt 2 werden ca. 30 Sekunden lang innig gemischt.

### Schritt 4:

Die Mischung aus Schritt 3 wird in eine Form gegossen und man lässt für ca. 1 h ausreagieren.

### Schritt 5:

Der gelierte Formkörper wird schockgefroren und gefriergetrocknet.

### Schritt 6:

Der gefriergetrocknete, großformatige, poröse bzw. schwammartige, gegebenenfalls mit zusätzlichen Stoffen beladene Formkörper kann wie oben erläutert konfektioniert werden.

## Patentansprüche

1. Schichtförmiger gefriergetrockneter poröser Formkörper, dessen Länge und Breite wenigstens 10-mal so groß ist wie dessen Dicke sind, enthaltend Alginat, Calcium, Sulfat sowie
Carboxymethylcellulose und/oder Hyaluronsäure und/oder deren Salze, der **dadurch gekennzeichnet ist, dass** er durch ein Verfahren erhältlich ist, umfassend das Mischen einer wässrigen Alginatlösung mit Calciumsulfat in Gegenwart mindestens einer Mineralsäure,
Giessen der erhaltenen Mischung in eine Form und
trocknen der Mischung.

2. Schichtförmiger gefriergetrockneter poröser Formkörper nach Anspruch 1, der beim suspendieren von 1 g des Formkörpers in 100g Wasser bei 20 °C einen pH-Wert der wässrigen Phase von weniger als 7 ergibt.

3. Schichtförmiger gefriergetrockneter poröser Formkörper nach einem der vorhergehenden Ansprüche, der eine Dichte von 0,005 bis 1 g/cm³ nach DIN 53420 aufweist.

4. Schichtförmiger gefriergetrockneter poröser Formkörper nach einem der vorhergehenden Ansprüche, der eine Nassreißfestigkeit nach DIN 53328 von mindestens 10 mN/mm Schichtdicke aufweist.

5. Schichtförmiger gefriergetrockneter poröser Formkörper nach einem der vorhergehenden Ansprüche, der zusätzlich mindestens eine weitere Komponente enthält, die ausgewählt ist aus der Gruppe die besteht aus: kosmetischen oder medizinischen Wirkstoffen, weiteren natürlichen oder synthetischen Hydrokolloid-bildenden Polymeren und kosmetischen oder medizinischen Hilfs- bzw. Zusatzstoffen.

6. Schichtförmlger gefriergetrockneter poröser Formkörper nach einem der vorhergehenden Ansprüche, enthaltend mindestens eine Hydroxycarbonsäure.

7. Schichtförmiger gefriergetrockneter poröser Formkörper nach einem der vorhergehenden Ansprüche, enthaltend mindestens einen kosmetischen und/oder pharmazeutischen Wirkstoff

8. Schichtförmiger gefriergetrockneter poröser Formkörper nach einem der vorhergehenden Ansprüche der eine Dicke, definiert als der kürzeste Abstand zwischen 2 Punkten, von mindestens 2 cm aufweist.

9. Schichtförmiger gefriergetrockneter poröser Formkörper nach einem der vorhergehenden Ansprüche, der in Form einer Schicht, Auflage kosmetische Maske oder Gesichtsmaske mit einer Schichtdicke von 0,5 bis 20 mm, vorliegt.

10. Schichtförmiger gefriergetrockneter poröser Formkörper nach Anspruch 9, der auf mindestens einer Seite mit mindestens einer Trägerschicht laminiert ist.

11. Schichtförmiger gefriergetrockneter poröser Formkörper nach einem der vorhergehenden Ansprüche zur Verwendung als pharmazeutisches Mittel.

12. Schichtförmiger gefriergetrockneter poröser Formkörper nach einem der vorhergehenden Ansprüche zur Verwendung als Wundauflage, transdermale Auflage, Wundpflaster, Wirkstoffpflaster, Mittel für die Verabreichung von Wirkstoffen, oral verabreichbares Mittel zur Wirkstofffreisetzung oder als Implantate.

13. Kombination, enthaltend mindestens einen schichtförmigen gefriergetrockneten porösen Formkörper nach einem der vorhergehenden Ansprüche sowie mindestens eine wässrige Lösung, die einen oder mehrere Wirkstoffe und/oder Hilfsstoffe enthält, in zusammengehöriger, räumlicher Anordnung.

14. Verwendung von schichtförmigen gefriergetrockneten porösen Formkörpern nach einem der vorhergehenden Ansprüche oder der Kombination nach Anspruch 1 bis 3 als kosmetisches Mittel, insbesondere als kosmetische Auflage, kosmetische Maske oder Gesichtsmaske, oder als Substrat zur Züchtung von Zellen.

## Claims

1. Layered freeze-dried porous shaped moulding, the length and width of which are at least 10 times greater than the thickness thereof, comprising alginate, calcium, sulfate and also carboxymethylcellulose and/or hyaluronic acid and/or salts thereof, **characterized in that** the said moulding is obtainable by a process which comprises mixing an aqueous alginate solution with calcium sulfate in the presence of at least one mineral acid, pouring the resulting mixture into a mould and drying the mixture.

2. Layered freeze-dried porous shaped moulding according to Claim 1 which, on suspending 1 g of the shaped moulding in 100 g of water at 20°C, results in a pH of the aqueous phase of less than 7.

3. Layered freeze-dried porous shaped moulding according to either of the preceding claims, having a density of 0.005 to 1 g/cm³ in accordance with DIN 53420.

4. Layered freeze-dried porous shaped moulding according to any of the preceding claims, having a wet tensile strength in accordance with DIN 53328 of at least 10 mN/mm of layer thickness.

5. Layered freeze-dried porous shaped moulding according to any of the preceding claims, additionally comprising at least one further component selected from the group consisting of: cosmetic or medicinal active ingredients, other natural or synthetic hydrocolloid-forming polymers and cosmetic or medicinal auxiliaries and additives.

6. Layered freeze-dried porous shaped moulding according to any of the preceding claims, comprising at least one hydroxycarboxylic acid.

7. Layered freeze-dried porous shaped moulding according to any of the preceding claims, comprising at least one cosmetic and/or pharmaceutical active ingredient.

8. Layered freeze-dried porous shaped moulding according to any of the preceding claims, having a thickness, defined as the shortest distance between two points, of at least 2 cm.

9. Layered freeze-dried porous shaped moulding according to any of the preceding claims, which is in the form of a layer, pad, cosmetic mask or face mask, having a layer thickness of 0.5 to 20 mm.

10. Layered freeze-dried porous shaped moulding according to Claim 9, which is laminated on at least one side with at least one support layer.

11. Layered freeze-dried porous shaped moulding according to any of the preceding claims for use as a pharmaceutical composition.

12. Layered freeze-dried porous shaped moulding according to any of the preceding claims for use as a wound dressing, transdermal patch, wound plaster, active ingredient patch, composition for administering active ingredients, composition for active ingredient release that can be administered orally or as an implant.

13. Combination comprising at least one layered freeze-dried porous shaped moulding according to any of the preceding claims and at least one aqueous solution comprising one or more active ingredients and/or auxiliaries in a spatially related configuration.

14. Use of layered freeze-dried porous shaped mouldings according to any of the preceding claims or the combination according to Claim 1 to 3 as a cosmetic composition, in particular as a cosmetic pad, cosmetic mask or face mask, or as a substrate for culturing cells.

## Revendications

1. Corps moulé poreux lyophilisé lamellaire, dont la longueur et la largeur sont au moins 10 fois plus grandes que son épaisseur, contenant un alginate, du calcium, un sulfate, ainsi que de la carboxyméthylcellulose et/ou de l'acide hyaluronique et/ou ses sels, qui est **caractérisé en ce qu'**il peut être obtenu par un procédé comprenant :
le mélange d'une solution aqueuse d'alginate avec du sulfate de calcium en présence d'au moins un acide minéral,
le versement du mélange obtenu dans un moule, et
le séchage du mélange.

2. Corps moulé poreux lyophilisé lamellaire selon la revendication 1, qui résulte lors de la suspension de 1 g du corps moulé dans 100 g d'eau à 20 °C en un pH de la phase aqueuse de moins de 7.

3. Corps moulé poreux lyophilisé lamellaire selon l'une quelconque des revendications précédentes, qui présente une densité de 0,005 à 1 g/cm³ selon DIN 53420.

4. Corps moulé poreux lyophilisé lamellaire selon l'une quelconque des revendications précédentes, qui présente une résistance à la déchirure à l'état humide selon DIN 53328 d'au moins 10 mN/mm d'épaisseur de couche.

5. Corps moulé poreux lyophilisé lamellaire selon l'une quelconque des revendications précédentes, qui contient en outre au moins un autre composant, qui est choisi dans le groupe constitué par : les agents actifs cosmétiques ou médicinaux, d'autres polymères naturels ou synthétiques formant des hydrocolloïdes, et les adjuvants ou additifs cosmétiques ou médicinaux.

6. Corps moulé poreux lyophilisé lamellaire selon l'une quelconque des revendications précédentes, contenant au moins un acide hydroxycarboxylique.

7. Corps moulé poreux lyophilisé lamellaire selon l'une quelconque des revendications précédentes, contenant au moins un agent actif cosmétique et/ou pharmaceutique.

8. Corps moulé poreux lyophilisé lamellaire selon l'une quelconque des revendications précédentes, qui présente une épaisseur, définie comme la distance la plus courte entre 2 points, d'au moins 2 cm.

9. Corps moulé poreux lyophilisé lamellaire selon l'une quelconque des revendications précédentes, qui se présente sous la forme d'une couche, d'un revêtement, d'un masque cosmétique ou d'un masque pour le visage d'une épaisseur de couche de 0,5 à 20 mm.

10. Corps moulé poreux lyophilisé lamellaire selon la revendication 9, qui est stratifié sur au moins un côté avec au moins une couche support.

11. Corps moulé poreux lyophilisé lamellaire selon l'une quelconque des revendications précédentes, destiné à une utilisation en tant qu'agent pharmaceutique.

12. Corps moulé poreux lyophilisé lamellaire selon l'une quelconque des revendications précédentes, destiné à une utilisation en tant que revêtement de plaie, revêtement transdermique, pansement de plaie, pansement à agent actif, agent pour l'administration d'agents actifs, agent administrable par voie orale pour la libération d'agents actifs, ou en tant qu'implant.

13. Combinaison, contenant au moins un corps moulé poreux lyophilisé lamellaire selon l'une quelconque des revendications précédentes, ainsi qu'au moins une solution aqueuse, qui contient un ou plusieurs agents actifs et/ou adjuvants, selon un agencement spatial formant un ensemble.

14. Utilisation de corps moulés poreux lyophilisés lamellaires selon l'une quelconque des revendications précédentes ou de la combinaison selon les revendications 1 à 3 en tant qu'agent cosmétique, notamment en tant que revêtement cosmétique, masque cosmétique ou masque pour le visage, ou en tant que substrat pour la culture de cellules.
